# EUROPEAN PATENT SPECIFICATION

(11) **EP 0 759 298 B1**
(45) Date of publication and mention of the grant of the patent: **05.11.2003**
(21) Application number: 96113501.9
(22) Date of filing: 22.08.1996
(51) Int. Cl.: A61K 31/519, A61P 9/12

(54) **Use of 4-amino-6-hydroxypyrazolo[3,4-d]pyrimidine for the manufacture of an antihypertensive agent**
Verwendung von 4-Amino-6-hydroxypyrazolo[3,4-d]pyrimidin zur Herstellung eines blutdrucksenkenden Mittels
Utilisation de 4-amino-6-hydroxypyrazolo[3,4-d]pyrimidine pour la préparation d'un agent antihypertensif

(30) Priority: 22.08.1995 JP 21329295
(43) Date of publication of application: 26.02.1997
(73) Proprietor: Maeda, Hiroshi, Dr., Kumamoto-shi, Kumamoto (JP)
(72) Inventor: Maeda, Hiroshi, Kumamoto-shi, Kumamoto (JP); Akaike, Takaaki, Kumamoto-shi, Kumamoto (JP); Miyamoto, Yoichi, Yokohama-shi, Kanagawa (JP); Yoshida, Masaki, Kumamoto-shi, Kumamoto (JP)
(74) Representative: DIEHL GLAESER HILTL & PARTNER

(56) References cited:
- WO-A-95/10185
- US-A- 3 474 098
- MIYAMOTO, YOICHI ET AL: "Potentiation of nitric oxide-mediated vasorelaxation by xanthine oxidase inhibitors" PROC. SOC. EXP. BIOL. MED., VOL. 211(4)1996, PP.366-73, XP002041196
- ELION, GERTRUDE B. ET AL: "Effects of xanthine oxidase inhibitors on purine catabolism" ISRAEL J. CHEM., VOL. 6(5), 1968, PP.787-96, XP002041197
- NAKAZONO K ET AL: "DOES SUPEROXIDE UNDERLIE THE PATHOGENESIS OF HYPERTENSION?" PROC NATL ACAD SCI U S A, 88 (22). 1991. 10045-10048., XP002041198
- OHARA, Y. ET AL: "Hypercholesterolemia increases endothelial superoxide anion production" THE JOURNAL OF CLINICAL INVESTIGATION, vol. 91, no. 6, June 1993, pages 2546-2551, XP002041199
- SUZUKI H. ET AL: "In vivo evidence for microvascular oxidative stress in spontaneously hypertensive rats: Hydroethidine microfluorography" HYPERTENSION, 1995, 25/5 (1083-1089), USA, XP002041200

## Description

The present invention relates to the use of 4-amino-6-hydroxopyrazolo[3,4-d]pyrimidine for the manufacture of a medicament for the treatment of hypertension.

Hypertension is generally considered as a risk factor of cardiac and circulatory diseases when either the diastolic blood pressure is higher than 90 mmHg or the systolic blood pressure is higher than 140 mmHg. Medical intervention with antihypertension drugs is often introduced when the modified daily life style does not work or fails to lower the blood pressure or when there is diagnosed a serious hypertension. Control of the blood pressure by treatment with a therapeutic agent is extremely important to reduce the possible complication of hypertension in the heart, the brain and the kidney. Antihypertensive agents utilized for this purpose are classified mainly to the following four groups based on their difference of action in their blood pressure lowering mechanisms: Uretics, sympathetic neuronal inhibitors, vasodilators such as calcium antagonists, and angiotensin converting enzyme inhibitors called ACE inhibitors.

Application of antihypertensive drugs with a moderate and reliable activity is generally recommended for the treatment of the hypertensive patients in clinical practice and special care should be taken in order to avoid rapid decline of blood pressure as to cause maladies in the patient's daily activity. Therefore, moderately effective antihypertensive agents are of great demand, which are capable of decreasing the blood pressure slowly.

Furthermore, in case the efficacy of a given antihypertensive agent is insufficient by increasing the dose of administration to some extent, further increase of dosing of the said agent is not advantageous usually and thus not recommended. However, a combination of such an agent with another type of antihypertensive drugs with a different mode of action is therefore preferred rather than simply escalating the dosing of a single agent because better efficacy and fewer side effects are usually expected in the combination therapy. Thus the development of an antihypertensive agent with a novel mechanism of action will provide a better therapeutic alternative for such a clinical setting.

From Israel J. Chem., Vo1. 6(5) 1968, pages 787-796, it was known that pyrazolo[3,4-d]pyrimidines, in particular 4-amino-6-hydroxypyrazolo[3,4-d]pyrimidine, are xanthine oxidase inhibitors. However, there is no hint in that document that this particular compound may be useful in the treatment of hypertension.

Accordingly, the present invention is directed to provide an antihypertensive agent with a different mode of action over the currently available antihypertensive agents, namely by use of a xanthin oxidase inhibitor. The results show a mild and reliable blood pressure lowering activity.

It is reported that endothelium derived relaxing factor (EDRF), an endogenous blood pressure lowering substance, which dilates the vascular wall by activating guanylate cyclase present in the smooth muscle cells of the blood vessel (Pharma. Rev., 43, 109-142, 1991). It is also reported that the endothelium derived relaxing factor is now identified as nitric oxide (NO), and thus NO is inactivated extremely rapidly by the reaction with superoxide radicals generated in situ (Nature, 320, 454-456, 1986; PNAS, 93, 2248-2253, 1996; J. Biol. Chem., 266, 4244-4250, 1991).

Among a class of compounds which are analogous to purine bases, like pyrazolopyrimidine derivatives, allopurinol is currently the agent of choice and commonly used for the treatment of gout and hyper-uricemia. Another pyrazolopyrimidine derivative, 4-amino-6-hydroxypyrazolo[3,4-d]pyrimidine (AHPP), is reported to be a potent inhibitor of three major purine metabolic enzymes, namely xanthine oxidase (J. Biol. Chem., 226. 993-1000, 1957), xanthine dehydrogenase (Yokohama Med. Bull., 29, 53-58, 1978) and tryptophan pyrolase (Life Sci., 8, 843-851, 1969).

The inventors of the present invention have perceived the inhibitory effect of AHPP against xanthine oxidase, and discovered a novel activity of AHPP to reduce blood pressure by enhancing EDRF due to decreased superoxide radical generation, more specifically by a novel mode of action by inhibiting the interaction of EDRF (which is now identified as nitric oxide) and superoxide (O₂⁻). The latter consumes NO by a rapid reaction. Medication containing AHPP can suppress the xanthine oxidase, which, as a result, leaves NO unreacted and at a high level to dilate blood vessels. Thus AHPP will function as an effective component for the treatment of hypertension and lower blood pressure with a novel action mechanism, and will provide an opportunity to develop an alternative therapeutic choice of agents for the treatment of hypertension.

The present invention is directed to the use of 4-amino-6-hydroxypyrazolo[3,4-d]pyrimidine of the following formula 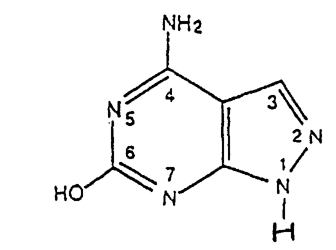 for the manufacture of a medicament for the treatment of hypertension.

AHPP can be chemically synthesized by reaction of 3-amino-43-amino-4-cyanopyrazole and urea (J. Am. Chem. Soc., 78, 784-790. 1956).

According to the present invention AHPP can be administered either orally or parenterally in form of the pure compound or a composite containing the compound of an appropriate dose, which is effective but without toxicity.

For oral administration of AHPP, the effective antihypertensive amount is 100-9000 mg/day/adult patient and can be taken once or more times in fractions divided, though the quantity of the compound will vary depending on the age, body weight and severity of the hypertension to be treated. For parenteral administration, intravenous injection is recommended but drip infusion may be performed.

For oral administration the compound can be formulated into solid or liquid preparations such as capsules, pills, tablets, powders, solutions, suspensions, liposomes, emulsions etc. by use of the standard procedures. The solid unit dosage forms are those as generally employed such as capsules or tablets. For parenteral administration the compound can be formulated as injectable dosage of a solution or suspension of the compound in a physiologically acceptable diluent as a pharmaceutical carrier vehicle under the condition utilized for the standard procedure of parenteral preparation. It may be used as aqueous solution of 0.001-5.0% (w/w) for infusion.

Injectable formulations may be also prepared as separate units in tightly sealed vials or ampoules, one of them contains the compound in powder, and the other vial or ampoule contains diluents for dissolving the compound. Upon usage, said powder is dissolved in the diluents.

The use according to the present invention not only achieves a mild and reliable blood pressure lowering activity, but also has a different mode of action over the currently available antihypertensive drugs. Therefore, improvement of efficacy and reduction of side effects is expected by administrating AHPP combined with another type of antihypertensive agents with a different mode of action.

### Examples

The present invention will now be described in detail with reference to the following examples.

### Procedure of Synthesis

### Example 1: Preparation of 3-amino-4-cyanopyrazole

To the 85% hydrazinehydrate solution of 3.4 g (57.8 mmole) were added 2.5 g (20.5 mmole) of ethoxymethylene-malononitril slowly, and further added the same amount (2.5 g). After cooling with cold water and heating to 90-100 °C for 1 hour, the reaction mixture yielded a solid reaction product. The reaction mixture was mixed with 3.3 ml of water and allowed to stand at 4 °C for 12 hours, the resulting yellow-brown solid reaction product (3.0 g in dry weight) was filtered and washed with 1.5 ml of water and recrystallized from 6.0 ml of water, which yielded 2.5 g of 3-amino-4-cyanopyrazole.

### Example 2: 4-amino-6-hydroxypyrazolo(3,4-d)pyrimidine

1.08 g of 3-amino-4-cyanopyrazole was mixed with 2.16 g of urea and heated at 200 °C for 20 minutes. The resulting solid substance was dissolved in 33 ml of 2N NaOH and the solution was filtered after heating to 90-100°C for 10 minutes after mixing with active charcoal powder. The filtrate was mixed with 6 ml of anhydrous acetic acid yielding 1.3 g of crystals after filtration. 0.6 g of the crystals of 4-amino-6-pyrazolo(3,4-d)pyrimidine (AHPP) was obtained by washing the crystals with a mixed solution of formamide/dimethylformamide (2/1, v/v), formamide and acetone, successively.
Physicochemical properties of thus obtained AHPP
- UV absorption: UV (λ max): at pH 11.0 : 270 nm
at pH 1.0: 250 nm
- Melting point:: 320°C

| Elementary analysis: | | | |
|---|---|---|---|
| theorical values: | C: 39.80, | H: 3.30, | N: 46.40 |
| experimental results: | C: 38.60, | H: 3.27. | N: 44.75 |

Fast Atom Bombardment Mass Spectrometry
[FAB-MS (Neg)]: [M-H]-: 150.00

### Formulations

### Example 3: Preparation of Tablets of AHPP

AHPP, lactose and starch were mixed and moistured granules are prepared by addition of polyvinylpyrrolidone solution and magnesium stearate. A preparation of 500 mg/tablet was obtained by a pressure tablet extruder with magnesium stearate, followed by drying. A representative weight ratio of the component of the tablet ingredients is

| | |
|---|---|
| AHPP | 100 |
| lactose | 235 |
| starch | 50 |
| 10% polyvinylpyrrolidone solution | 50 |
| magnesium stearate | 3 |

### Example 4: Preparation of Suspension Syrup of AHPP

1.5 of sorbitol and 0.005 g of glycerol were dissolved in 3.0 l of water, and the solution was mixed with a portion of a benzoic acid water solution prepared separately. A representative ratio of the components used was as follows:

| | |
|---|---|
| AHPP | 25 g |
| sorbitol | 1.5 g |
| glycerol | 0.005 g |
| dispersion type carboxy methyl cellulose | 0.005 g |
| water filled up to | 5 l |

### Example 5: Inhibitory Effect of AHPP Against Uric Acid Production by Xanthine Oxidase In Vitro

The rate of uric acid production was determined by measuring the increase of an optical absorbance at 230 nm in 50 mM phosphate buffer solution (pH 7.5) containing 2.19-21.9µM of xanthine and 0-0.44µM AHPP in the presence of cow milk xanthine oxidase in the final concentration of 3.33 mU/ml. The results showed that uric acid production by xanthine oxidase was inhibited by AHPP in a dose dependent manner. The inhibitory coefficient (Ki) of AKPP for xanthine oxidase was determined to be 0.2 µM by an analysis of its reaction velocity kinetics and the inhibitory of action of AHPP was also demonstrated as a competitive inhibition.

### Experiments of Biochemical and Biological Effects.

### Example 6: Inhibitory Effect of AHPP against Superoxide Production by Xanthine Oxidase In Vitro

Production of superoxide radicals was determined by measuring a chemiluminescence observed in 100mM phosphate buffer (pH 7.4.) containing 10µM lucigenin and 10µM xanthine in the presence of 20 U/ml of xanthine oxidase derived from cow milk, and inhibition of superoxide generation by AHPP was evaluated by addition of 0-100µM of AHPP into the reaction mixture. AHPP was shown to inhibit superoxide production by xanthine oxidase in dose-dependent manner. The results are shown in Table 1.

**Table 1**

| Effect of AHPP to inhibit superoxide generation by xanthine oxidase with hypoxanthine | |
|---|---|
| Concentration of AHPP(µM) | Integral value of intensity of chemiluminescence/10 min ∗ |
| 0 | 5.2 × 10⁶ count |
| 1 | 4.4 × 10⁶ count |
| 10 | 1.6 × 10⁶ count |
| 100 | 8.0 × 10⁴ count |

| | |
|---|---|
| ∗ Average of two measurements | |

### Example 7: Synergistic Vascular Relaxation Effect of AHPP to Acetylcholine in Case of a Rabbit Aortic Ring by AHPP

Aortic rings with a diameter of 5 mm were. prepared from the chest aortas of New Zealand White female rabbits with a body weight of 2.5-3.0 kg. The rings were surgically prepared and hang into the organ bath containing 20 ml of Krebs solution, which was kept at 37°C and supplemented with 95% O₂ and 5% CO₂, for determination of the tension. The aortic ring relaxation by 0-1µM acetylcholine was measured in the presence of 0, 100, and 300µM of AHPP, after the aortic ring contraction was induced by 0.15µM phenylepherine. As shown in Table 2, the dose of phenylepherine required for 50% relaxation of phenylepherine-induced aortic ring contraction is reduced depending on the concentration of AHPP present in the solution.

**Table 2**

| Effect of AHPP on the inhibition of vascular relaxation by acetylcholine | |
|---|---|
| Concentration of AHPP(µM) | Amount of acetylcholine required to inhibit 50% relaxation of aorta ring ∗ |
| 0 | 0.11 |
| 100 | 0.075 |
| 300 | 0.055 |

| | |
|---|---|
| ∗ Average of four measurements | |

### Example 8: Effect of Intravenous Injection of AHPP on the Blood Pressure of Spontaneously Hypertensive Rats

Eighteen weeks old spontaneously hypertensive male rats weighing 300-400 g were intravenously infused with 50 mg/kg of AHPP in a solution in 1 ml of 0.1M NaOH for 5 minutes (8 rats). The mean arterial blood pressure was measured under anesthesia. Eight control rats received only 0.1 M NaOH by intravenous infusion. The mean arterial blood pressure of the treated groups showed a gradual decrease up to 72% of the value before the treatment 70 minutes after the AHPP treatment. No such drop of blood pressure was evident in the untreated control group. The results are shown in Table 3.

**Table 3**

| Hypotensive effect of AHPP in spontaneous hypertensive rats (values are average of 8 rats ± SE). | | |
|---|---|---|
| Time (min) after iv injection of AHPP | Mean arterial blood pressure: 100%=preinjection value | |
| | AHPP group | Control (vehicle only) |
| 0 | 100 | 100. |
| 5 | 98.3 ± 2.7 | 105.5 ± 7.5 |
| 10 | 92.2 ± 6. 1 | 107.3 ± 6.9 |
| 15 | 91.1 ± 5.6 | 106.1 ± 6. 3 |
| 20 | 85.9 ± 8.0 | 105.0 ± 6.6 |
| 30 | 81.7 ± 5.0 | 103.3 ± 4.2 |
| 40 | 77.7 ± 5.2 | 100.5 ± 3.1 |
| 50 | 76.1 ± 7.2 | 98.9 ± 3.3 |
| 60 | 73.5 ± 7.5 | 97.5 ± 4.1 |
| 70 | 72.4 ± 6. 6 | 96.7 ± 5.0 |

Dose of AHPP: 50 mg/kg in rats, 0.33 mmol/ml, 1.0 ml/5 min. See text for details. Average value of 8 measurements ± SD.

### Example 9: Effect of Oral Administration of AHPP on the Blood Pressure of Spontaneously Hypertensive Rats

Four male spontaneously hypertensive rats with a body weight of 300-400 g and 4 male rats with a body weight of 200-300 g were orally treated with 100 mg/kg of AHPP. The systolic blood pressure in the tail artery was examined 4 hours after the treatment. The systolic blood pressure of the treated group was reduced to 70% of the value before the treatment.

## Claims

1. Use of 4-amino-6-hydroxypyrazolo[3,4-d]pyrimidine according to the following formula 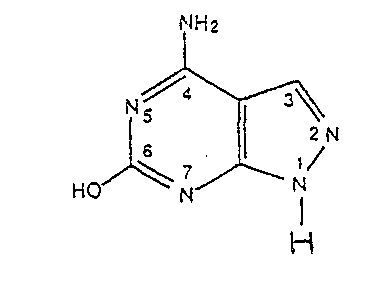 for the manufacture of a medicament for the treatment of hypertension.

## Patentansprüche

1. Verwendung von 4-Amino-6-hydroxypyrazolo[3,4-d] pyrimidin gemäß der folgenden Formel 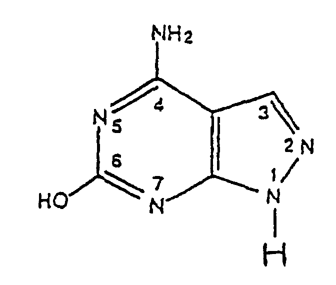 zur Herstellung eines Medikaments zur Behandlung von Bluthochdruck.

## Revendications

1. Utilisation de 4-amino-6-hydroxypyrazolo[3,4-d]pyrimidine selon la formule suivante 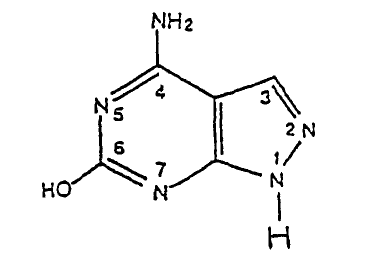 pour la préparation d'un médicament pour le traitement de l'hypertension.
